# EUROPEAN PATENT APPLICATION

(11) **EP 4 245 356 A1**
(43) Date of publication of application: **20.09.2023**
(21) Application number: 22162196.4
(22) Date of filing: 15.03.2022
(51) Int. Cl.: A61M 60/816, A61M 60/174

(54) **SENSOR CARRIER, BLOOD PUMP COMPRISING A SENSOR CARRIER AND GUIDEWIRE COMPRISING A SENSOR CARRIER**

(71) Applicant: Abiomed Europe GmbH, 52074 Aachen (DE)
(72) Inventor: The designation of the inventor has not yet been filed
(74) Representative: Wittmer, Maximilian

(57) **Abstract**

The present invention relates to a sensor carrier 10 configured to be attached to an elongated medical device. The sensor carrier comprises a sensor 12 having a sensor longitudinal axis SLA, a base member 14, 314 having a central longitudinal axis CLA and an attachment portion 16 for receiving the sensor 12, the sensor 12 being configured to be disposed in the attachment portion 16, wherein the sensor longitudinal axis SLA is non-parallel with respect to the central longitudinal axis CLA of the base member 14. The present invention further relates to a blood pump or a guidewire comprising a sensor carrier.

## Description

### BACKGROUND

The present invention relates to a sensor carrier configured to be attached to an elongated medical device, such as a blood pump or a guidewire. The present invention further relates to a blood pump comprising a sensor carrier and to a guidewire comprising a sensor carrier. The blood pump may be a catheter pump, in particular an intravascular blood pump, an intracardiac blood pump or any other kind of ventricular assist device.

Such blood pumps of different types are known from the prior art and are intended to support the function of a patient's heart, either in a short-term application, in which an intravascular blood pump is placed in the patient for a couple of days or weeks, or a long-term application, in which the intravascular blood pump is placed in the patient for a couple of weeks or months. The blood pumps may e.g. be inserted into a patient's body through the aorta by means of a catheter, or may be placed in the thoracic cavity. During planned surgery, intravascular blood pumps are often introduced into the patient's body along a guidewire. For example, guidewires and intravascular blood pumps are introduced into the patient's body using fluoroscopy to warrant a correct positioning of the intravascular blood pump in the patient's left ventricle. Commonly, X-ray is used for fluoroscopy.

However, there are certain disadvantages associated with fluoroscopy and in particular with X-ray. During planned surgery for placing the blood pump or the guidewire respectively X-ray is not available without restrictions. In addition, the patient and the medical staff are exposed to a high amount of radiation when conducting X-ray. When it comes to an emergency condition making it necessary to immediately introduce an intravascular blood pump into the patient's body, X-ray is often not available, e.g. at an Immediate Care Unit or in an ambulance car. Furthermore, during emergency conditions intravascular blood pumps are introduced into the patient's body without a guidewire, e.g. through a femoral access.

Therefore, the need exists to provide an alternative solution for safely introducing an elongated medical device, such as an intravascular blood pump or a guidewire, into a patient's body and to warrant correct positioning of the elongated medical device in the patient's heart.

### SUMMARY OF THE INVENTION

According to a first aspect, there is provided a sensor carrier configured to be attached to an elongated medical device. The sensor carrier comprises a sensor having a sensor longitudinal axis, a base member having a central longitudinal axis and an attachment portion for receiving the sensor. The sensor is configured to be disposed in the attachment portion, wherein the sensor longitudinal axis is non-parallel with respect to the central longitudinal axis of the base member. Preferably, the sensor is an embedded electromagnetic sensor, in particular having a longish shape, preferably a cylindrical shape.

The position of the sensor within the patient's body can be tracked by suitable means, e.g. by a field generator and at least one reference sensor. Generally, sensors with the X-direction orientated in parallel with the movement direction allow for detection of motions with five degrees of freedom (5DOF), namely along the X-axis, the Y-axis and the Z-axis, as well as rotations around the Y-axis and the Z-axis. Here, the movement direction coincides with the central longitudinal axis of the base member. When the sensor longitudinal axis is non-parallel with respect to the central longitudinal axis of the base member, this allows for detection of a further motion, namely rotation around the X-axis. That is, the sensor longitudinal axis is inclined with respect to the central longitudinal axis of the base member. This results in a sensor with five degrees of freedom (5DOF) additionally offering the possibility of position measurement. This is particularly of advantage in case the sensor is used with an elongated medical device, which desires further for a correct positioning around the X-axis. In addition, such elongated medical devices usually have limited space to add a further five degrees of freedom sensor to obtain a six degrees of freed om sensor (6DOF).

Preferably, the sensor longitudinal axis intersects with the central longitudinal axis of the base member. This minimizes the need to apply spatial corrections, as the sensor is not radially displaced with respect to the central longitudinal axis of the base member.

Preferably, the base member comprises a magnetically permeable material or is composed of a magnetically permeable material. Using magnetically permeable material hinders shadowing of an electromagnetic field inducing the current in the sensor for tracking the position thereof, e.g. by a field generator placed in vicinity to the patient's body establishing a measurement volume. Preferably, the base member is composed of a ceramic material or a plastic material, in particular of a polyaryletherketone such as polyether ether ketone (PEEK). This further allows for a high biocompatibility and eases manufacturing of the sensor carrier.

Preferably, the base member comprises an axial through hole concentric with the central longitudinal axis, wherein the axial through hole preferably comprises the attachment portion. Some elongated medical devices require an axial through hole for the medical device to be guided along e.g. a guidewire. Hence, the sensor can easily be installed in an existing structure.

Preferably, the sensor carrier is a teardrop of a blood pump, preferably of an intravascular blood pump. Preferably, the sensor carrier further comprises an elastic extension with a pigtail having a tip. The sensor longitudinal axis preferably points to the tip of the pigtail, when the pigtail is in a reeled state. Hence, the tip of the pigtail can be marked allowing for a correct positioning of the pigtail within the patient's vascular system, e.g. a smoothly rounded portion of the pigtail may be orientated to abut the inner surface of the left ventricle .

Preferably, the elastic extension comprises an opening for receiving a guidewire. The guidewire is configured to elastically reel and unreel the pigtail, when the guidewire is moved relative to the pigtail and the teardrop respectively. Before introducing an intravascular blood pump with an inventive sensor carrier and a pigtail into the blood vessel and further into the left ventricle, the pigtail needs to be unreeled to pass the introducer and the aortic valve. Further, the pig tail functions as spacer inhibiting suctioning of the intravascular blood pump at the surface of the heart, when installed.

Preferably, the opening of the elastic extension is radially spaced from the central longitudinal axis of the base member. This avoids interference of the guidewire with the sensor disposed in the attachment portion of the base member.

Preferably, the sensor carrier further comprises a coupling element attached to the base member. The coupling element is configured to be attached to a suction head disposed at a distal end of a cannula. Preferably, the coupling element is made of metallic material, such as stainless steel, titanium or platinum. Thus, it is possible to securely fix the sensor carrier to the suction head of the cannula, as metallic material has a high mechanical resistance. In addition, certain metallic materials have a high biocompatibility necessary for medical use.

Alternatively, the sensor carrier is configured to be attached to a guidewire for introducing a blood pump into a patient's body, preferably an intravascular blood pump. When the blood pump is to be installed during a scheduled surgery, the medical staff can easily track the position of the guidewire without the need to use fluoroscopy.

According to a second aspect, there is provided a blood pump comprising a sensor carrier according to the first aspect (as described above), a cannula and a suction head. The sensor carrier is coupled to the suction head. The blood pump is preferably an intravascular blood pump, but may also be an intracardiac blood pump or any other kind of ventricular assist device.

According to a third aspect, there is provided a blood pump comprising a sensor carrier including a sensor, a cannula and a suction head. The sensor carrier is coupled to the suction head. The blood pump is preferably an intravascular blood pump, but may also be an intracardiac blood pump or any other kind of ventricular assist device.

Preferably, at least one cable or at least one lead is connected to the sensor and the cable is guided along and/or within the cannula. The current induced in the sensor can be transmitted via the at least one cable to e.g. a controller to visualize the position of the sensor to the medical staff. Preferably, two cables or leads are provided. Preferably, the two cables or leads are twisted. Twisting the cables or leads reduces the signal noise, so that no additional shielding is required.

Preferably, the at least one cable is helically guided along and/or within the cannula. This avoids the at least one cable to be ripped during movement of the cannula, e.g. due to bending or twisting. Hence, it is warranted that the position of the sensor can be reliably tracked.

Preferably, the cannula further comprises a coiled stabilizing structure provided along and/or within the cannula, wherein the at least one cable is guided in-between the coiled stabilizing structure. In other words, the at least one cable is guided analogously to the coiled stabilizing structure and is further positioned between coils of the stabilizing structure. This further inhibits damage of the at least one cable due to movement of the cannula. Preferably, the coiled stabilizing structure is made of Nitinol.

### BRIEF DESCRIPTION OF THE DRAWINGS

The foregoing summary, as well as the following detailed description of exemplary embodiments, will be better understood when read in conjunction with the appended drawings. For the purpose of illustrating the present disclosure, reference is made to the drawings. The scope of the disclosure is not limited, however, to the specific embodiments disclosed in the drawings.

In the drawings:
- Fig. 1: is a schematic and partially sectional view of a patient's heart with an introduced intravascular blood pump;
- Fig. 2A: is schematic sideview of a sensor carrier;
- Fig. 2B: is a schematic back view of the sensor carrier of FIG: 2A;
- Fig. 3: is a schematic sectional view of a sensor carrier in form of a teardrop according to a first embodiment;
- Fig. 4: is a schematic sectional view of a sensor carrier in form of a teardrop according to a second embodiment;
- Fig. 5: is a schematic view of a cannula of a blood pump with a sensor without a suction head and sensor carrier; and
- Fig. 6: is a schematic view of a guidewire with a sensor carrier.

### DETAILED DESCRIPTION

Embodiments of the present disclosure are described in detail with reference to the figures wherein like reference numerals identify similar or identical elements. It is to be understood that the disclosed embodiments are merely examples of the disclosure, which may be embodied in various forms. Well known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

To provide an overall understanding of the systems, methods, and devices described herein, certain illustrative examples will be described. Although various examples may describe intravascular blood pumps, it will be understood that the improvements of the present technology may also be adapted and applied to other types of medical devices such as electrophysiology study and catheter ablation devices, angioplasty and stenting devices, angiographic catheters, peripherally inserted central catheters, central venous catheters, midline catheters, peripheral catheters, inferior vena cava filters, abdominal aortic aneurysm therapy devices, thrombectomy devices, TAVR delivery systems, cardiac therapy and cardiac assist devices, including balloon pumps, cardiac assist devices implanted using a surgical incision, and any other venous or arterial based introduced catheters and devices.

As is known, intravascular blood pumps can be introduced into a patient, either surgically or percutaneously, to deliver blood from one location in the heart or circulatory system to another location in the heart or circulatory system. For example, when deployed in the left ventricle, an intravascular blood pump can pump blood from the left ventricle of the heart into the aorta. When deployed in the right ventricle, an intravascular blood pump can pump blood from the inferior vena cava into the pulmonary artery.

Herein, "proximal" and "distal" are seen relative to the medical staff or physician. Thus, proximal designates something which is relatively close to the physician whereas distal designates something which is relatively far away from the physician when the elongated medical device is introduced into the patient's body.

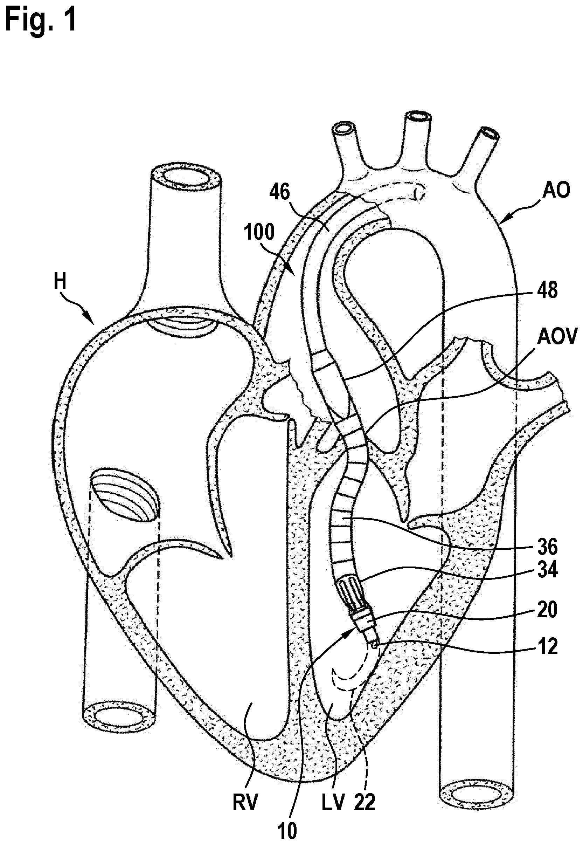

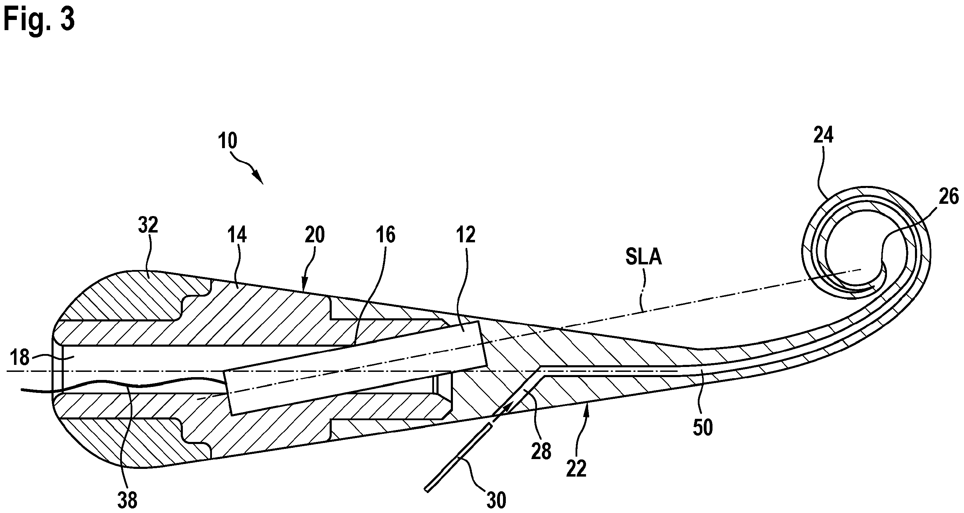

Fig. 1 shows the use of an elongated medical device. In this embodiment, the elongated medical device is an intravascular blood pump 100 for supporting, in this particular example, a left ventricle LV of a human heart H. The intravascular blood pump 100 comprises a catheter 46, a pump member 48, a cannula 36, a suction head 34, a sensor carrier 10 in form of a teardrop 20 and an elastic extension 22. Here, the elastic extension 22 is shown in dashed lines for lucidity reasons. The pump member 48 is disposed on a distal end of the catheter 46. The cannula 36 connects the pump member 48 with the suction head 34 and the sensor carrier 10 is fixed to the suction head. The elastic extension 22 is fixed to the sensor carrier 10 or teardrop 20 respectively and will be described in more detail below with reference to Fig. 3.

As one can take from Fig. 1, the blood pump 100 is partially disposed in the left ventricle LV, whereby the cannula 36 reaches through the aortic valve AOV into the left ventricle LV. The pump member 48 is disposed in the aorta AO and delivers blood from the suction head 34 and hence from the left ventricle LV to the aorta AO in a known manner.

The intravascular blood pump 100 may be placed inside the heart H using an inventive sensor carrier 10 as will be described in more detail below. For example, the intravascular blood pump 100 may be introduced through a femoral artery. However, alternative vascular access is equally possible, such as access through the subclavian artery or any other percutaneous access. The positioning of the intravascular blood pump 100 in Fig. 1 serves purely as an example, whereas different placements are possible, such as positioning the intravascular blood pump 100 inside the right ventricle RV of the heart H.

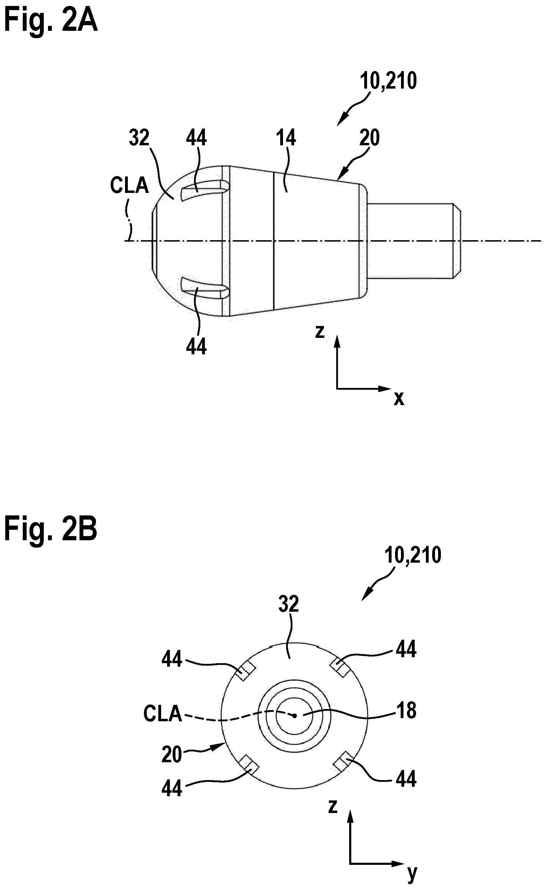

Figs. 2A and 2B show the sensor carrier 10 in more detail. The sensor carrier 10 comprises a base member 14 and a coupling element 32. In this embodiment, the sensor carrier 10 is a teardrop 20 of an intravascular blood pump. The base member 14 is composed of a non-metallic material with high magnetic permeability, such as ceramic or plastic. In this particular embodiment, the base member 14 is composed of polyether ether ketone (PEEK) offering a high biocompatibility. The coupling element 32 is attached to a proximal end of the base member 14, e.g. by press fitting. Of course, other fixing techniques are also possible, e.g. shrinking.

The coupling element 32 is composed of metallic material, such as stainless steel, titanium or platinum offering a high biocompatibility. The coupling element 32 comprises a plurality of coupling recesses 44 which are disposed on the outer surface of the coupling element 32. In the embodiment shown, there are four coupling recesses 44 evenly circumferentially distributed. The suction head 34 can be coupled to the sensor carrier 10 by means of the coupling recesses 44 in a known manner.

Further, sensor carrier 10 has a central longitudinal axis CLA extending axially through the sensor carrier 10. There is further provided an axial through hole 18 concentric with the central longitudinal axis CLA extending axially through the sensor carrier 10.

Fig. 3 shows a first exemplary embodiment of the sensor carrier 10 shown in Fig. 1 as a sectional view. The base member 14 comprises an attachment portion 16 configured to receive a sensor 12. A sensor 12 having a sensor longitudinal axis SLA is received in the attachment portion 16. As one can take from Fig. 3, the sensor 12 has a virtually cylindrical shape and, in this embodiment, it is an embedded electromagnetic sensor. The attachment portion 16 is inclined with respect to the axial through hole 18 and the central longitudinal axis CLA of the base member 14. Accordingly, the sensor 12 is not mounted in parallel to the axial through hole 18, but rather is also inclined with respect to the central longitudinal axis CLA of the base member 14. In particular, the sensor longitudinal axis SLA is non-parallel with the central longitudinal axis CLA of the base member 14 and intersects with the central longitudinal axis CLA of the base member 14. This non-parallel orientation of the sensor 12 allows for detection of motions of the sensor carrier with 6DOF, namely motion along the X-axis, the Y-axis and the Z-axis, as well as rotations around the X-axis, the Y-axis and the Z-axis (see Figs. 2A and 2B).

Further, the elastic extension 22 is fixed to the distal end of the base member 14 opposite the coupling element 32. The elastic extension 22 comprises a pigtail 24 having a tip 26. The elastic extension 22 comprises an opening 28 for receiving a guidewire (not shown) configured to elastically reel and unreel the pigtail 24. Here, the opening 28 merges into a channel 50 extending inside the pigtail 24 in a known manner. Furthermore, the elastic extension 22 is made of a highly magnetically permeable material, in particular of a plastic material, such as polyurethan, nylon, polyether block amide (pebax) or combinations thereof. Of course, the pigtail 24 can have a shape different from the shape shown in Fig. 3, e.g. a J-shape. Further, the pigtail 24 can have a different stiffness from its proximal portion to its distal portion, with a higher stiffness preferably at the proximal portion coupled to the base member 14. Of course, different designs of the pigtail 24 are possible, e.g. without an opening and/ or without a channel and/or with the channel being off- center of the pigtail.

As can be taken from Fig. 3, the sensor 12 is inclined in a pre-defined angle so that the sensor longitudinal axis SLA points towards the tip 26 of the pigtail 24 when the pigtail is reeled. This allows to easily track the position of the pigtail 24 when introducing the intravascular blood pump 100 into the patient's body, as the rounded portion of the pigtail can be correctly positioned to about the surface of the blood vessel the blood pump is fed through, e.g. the aorta.

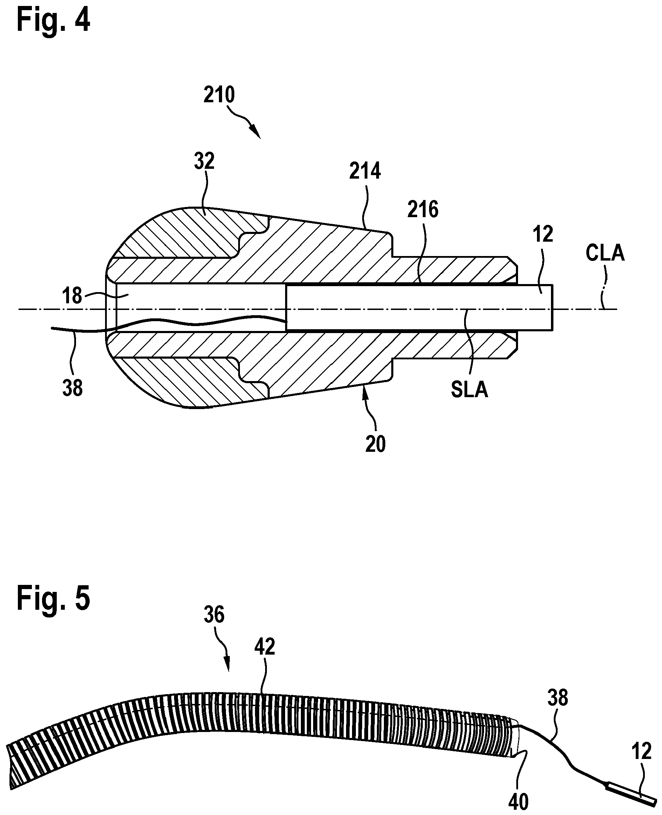

A cable 38 is attached to the proximal end of the sensor 12 which runs through the axial through hole 18. The cable 38 is further guided within the cannula 36 and connected to a controller located outside the patient's body. The current induced in the sensor 12 by a magnetic field generator is transferred by means of the cable 38 to track the sensor 12 within the patient's body. The specific guidance of the cable 38 within the cannular 36 will be described below in more detail with reference to Fig. 5.

Fig. 4 shows a second embodiment of a sensor carrier 210 to be used with an intravascular blood pump. The sensor carrier 210 according to the second embodiment differs from the sensor carrier 10 according to the first embodiment in the configuration of the base member 214. In particular, the attachment portion 216 is not inclined with respect to the axial though hole 18, but rather is disposed within the axial through hole 18. Hence, the sensor 12 is mounted directly within the axial through hole 18 and the sensor longitudinal axis SLA is concentric with the central longitudinal axis CLA of the base member 216. Of course, this only allows for a detection of motion of the sensor carrier 210 with 5DOF, but depending on the application it might not be necessary to detect rotation around the X-axis, e.g. in case no pigtail is used. Of course, the distal end of the base member 214 and the sensor 12 protruding therefrom in the axial direction can be covered by a suitable cap member.

Fig. 5 shows details of the cannula 36 and how the cable 38 is guided within and along the cannula 36. The cannula 36 is preferably composed of a polyurethane material and has a tube-like shape. A coiled stabilizing structure 42 is provided within the cannula 36 along an inner peripheral surface 40 of the cannula 36. The coiled stabilizing structure 42 is preferably composed of Nitinol and may have a further layer covering the Nitinol. The coiled stabilizing structure 42 stabilizes the cannula 36 when being pushed through the patient's blood vessels. As shown in Fig. 5, the coiled stabilizing structure is helically wound around the inner peripheral surface 40 of the cannula 36. The cable 38 attached to the sensor 12 is also guided along and within the cannula 36. In particular, the cable 38 is guided helically along the cannula 36 sandwiched between neighboring coils of the coiled stabilizing structure 42. Thus, bending of the cannula 36 during introduction of the intravascular blood pump into the patient's body does not lead to ripping of the cable 38 and hence, warrants a safe and stable tracking of the sensor 12.

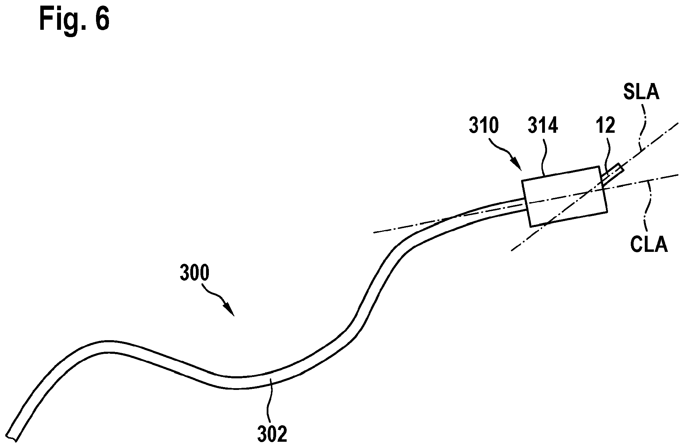

Fig. 6 shows an embodiment with a sensor carrier 310 attached to a guidewire 300 having a guidewire body 302. The guidewire 300 serves as a guide track for a blood pump to be introduced into a patient's body in a known manner. Hence, the guidewire 300 is firstly introduced into the patient's body and the blood pump is thereafter introduced by being guided along the guidewire 300. To warrant for a correct positioning of the guidewire 300, the sensor carrier 310 is attached to the distal end of the guidewire body 302.

The sensor carrier 310 comprises a base member 314 composed of a highly magnetically permeable material, such as a plastic material. In particular, the base member 314 is composed of polyether ether ketone (PEEK). The base member 314 comprises a central longitudinal axis CLA and the sensor 12 is embedded within a (not shown) attachment portion of the base member 314 so that a sensor longitudinal axis SLA is inclined with respect to the central longitudinal axis CLA. Hence, the sensor longitudinal axis SLA is non-parallel with respect to the central longitudinal axis CLA of the base member 314. Thus, the sensor 12 allows for detection of movement of the sensor carrier 310 with 6DOF, namely motion along the X-axis, the Y-axis and the Z-axis, as well as rotations around the X-axis, the Y-axis and the Z-axis.

The at least one cable connected to the sensor 12 is guided withing the guidewire body 302. Preferably, the at least one cable is embedded or casted in the guidewire body 302.

Next, real-time tracking of the sensor 12 with a patient's body will be described in more detail.

A field generator is placed in vicinity to the patient's body, preferably under the patient's body, e.g. embedded in a bed, a stretcher or an operating table. The field generator emits a varying electromagnetic field of low-intensity. Said electromagnetic field establishes a measurement volume in which the positions and orientations of the sensor 12 are tracked. The field generator is arranged relative to the patient's body so that the measurement volume covers the intended path of the sensor 12 within the patient's body.

The electromagnetic field induces a current within the sensor 12, which is relayed via the cable to a controller. Depending on the application, the current is amplified and digitalized by the controller in an appropriate way to generate a signal. Said signal is further processed to visualize the position of the sensor 12 (along the X-axis, the Z-axis and the Y-axis, as well as the rotational position relative to the axes) within the measurement volume. In particular, the position of the sensor 12 is visualized to the medical staff or physician on a display device.

To obtain real-time tracking with higher resolution and/or quality, a reference sensor can be placed on the patient's body within the electromagnetic field or measurement volume respectively. As the reference sensor moves together with patient's body, the position of the sensor 12 can also be tracked relative to the reference sensor, i.e. relative to the patient's body. Of course, appropriate adjustment of the coordinate system is required in this case.

### EXEMPLARY IMPLEMENTATIONS

As already described, the technology described herein may be implemented in various ways. In that regard, the foregoing disclosure is intended to include, but not be limited to, the systems, methods, and combinations and sub-combinations thereof that are set forth in the following exemplary implementations. Preferred embodiments are described in the following paragraphs:
A1 Sensor carrier configured to be attached to an elongated medical device comprising: a sensor having a sensor longitudinal axis, a base member having a central longitudinal axis and an attachment portion for receiving the sensor, the sensor being configured to be positioned in the attachment portion, wherein the sensor longitudinal axis is non-parallel with respect to the central longitudinal axis of the base member.
A2 Sensor carrier according to paragraph A1, wherein the sensor longitudinal axis of the sensor intersects with the central longitudinal axis of the base member.
A3 Sensor carrier according to paragraph A1 or A2, wherein the base member comprises a magnetically permeable material or is composed of a magnetically permeable material, in particular a plastic material.
A4 Sensor carrier according to paragraph A3, wherein the plastic material is PEEK (Polyether ether ketone).
A5 Sensor carrier according to any one of the preceding paragraphs A1 to A4, wherein the base member comprises an axial through hole concentric with the central longitudinal axis, and wherein the axial through hole comprises the attachment portion.
A6 Sensor carrier according to any one of the preceding paragraphs A1 to A5, wherein the sensor carrier is a teardrop of a blood pump, in particular of an intravascular blood pump.
A7 Sensor carrier according to paragraph A6, wherein the sensor carrier further comprises an elastic extension with a pigtail having a tip, wherein the sensor longitudinal axis points to the tip of the pigtail.
A8 Sensor carrier according to paragraph A7, wherein the elastic extension comprises an opening for receiving a guidewire, the guidewire being configured to elastically reel and unreel the pigtail.
A9 Sensor carrier according to paragraph A8, wherein the opening is radially spaced from the central longitudinal axis of the base member.
A10 Sensor carrier according to paragraph A8 or A9, wherein the opening merges into a channel extending through the pigtail.
A11 Sensor carrier according to any one of the preceding paragraphs A1 to A10, wherein the sensor carrier further comprises a coupling element attached to the base member, the coupling element being configured to be attached to a suction head disposed at a distal end of a cannula.
A12 Sensor carrier according to paragraph A11, wherein the coupling element is made of metallic material, in particular of stainless steel, titanium or platinum.
A13 Sensor carrier according to any one of the preceding paragraphs A1 to A3, wherein the sensor is a an embedded electromagnetic sensor, in particular having a longish shape.
A14 Sensor carrier according to any one of the preceding paragraphs A1 to A3, wherein the sensor carrier is configured to be attached to a guidewire for introducing a blood pump into a patient's body.
A15 Elongated medical device, preferably a blood pump, comprising: a sensor carrier according to any one of the preceding paragraphs A1 to A13, a cannula, and a suction head fixed to the cannula, wherein the sensor carrier is coupled to the suction head.
A16 Elongated medical device, preferably a blood pump comprising: a sensor carrier including a sensor, a cannula, and a suction head fixed to the cannula, wherein the sensor carrier is coupled to the suction head.
A17 Elongated medical device according to paragraph A15 or A16, wherein at least one cable is connected to the sensor.
A18 Elongated medical device according to any one of the preceding paragraphs A15 to A17, wherein the at least one cable or lead is guided along and/ or within the cannula.
A19 Elongated medical device according to paragraph A18, wherein the at least one cable or at least one lead is helically guided along and/or within the cannula.
A20 Elongated medical device according to paragraph A18 or A19, wherein the cannula further comprises a coiled stabilizing structure provided at the inner peripheral surface of the cannula, wherein the at least one cable is guided in-between the coiled stabilizing structure.
A21 Elongated medical device according to paragraph A20, wherein the coiled stabilizing structure is made of Nitinol.
A22 Guidewire for introducing a blood pump into a patient's body comprising a sensor carrier according to paragraph A14.
A23 Guidewire according to paragraph A22, wherein at least one cable or at least one lead is connected to the sensor.
A24 System comprising: an elongated medical device according to any one of the preceding paragraphs A17 to A21 and/ or a guidewire according to paragraph A23, a field generator and a controller connected to the at least one cable, the field generator being configured to emit an electromagnetic field, and the controller being configured to receive a current induced in the sensor, when the sensor moves within the electromagnetic field.
A25 System according to paragraph A24, the controller being further configured to process the current of the sensor, so that a signal is generated.
A26 System according to paragraph A25, wherein processing includes at least one of amplifying and digitalizing.
A27 System according to any one of the preceding paragraphs A25 or A26, the system further comprising a display device connected to the controller, the controller being further configured to visualize the real-time position of the sensor on the display device based on the signal.
A28 System according to any one of the preceding paragraphs A24 to A27, the system further comprising a reference sensor being configured to be positioned on a patient's body.

### List of reference signs

- 10, 210, 310: sensor carrier
- 12: sensor
- 14, 314: base member
- 16: attachment portion
- 18: axial through hole
- 20: teardrop
- 22: elastic extension
- 24: pigtail
- 26: tip of pigtail
- 28: opening
- 30: guidewire
- 32: coupling element
- 34: suction head
- 36: cannula
- 38: cable
- 40: inner peripheral surface of cannula
- 42: coiled stabilizing structure
- 44: coupling recess

- AO: aorta
- AOV: aortic valve
- CLA: central longitudinal axis of base member
- LV: left vascular
- RV: right vascular
- SLA: sensor longitudinal axis

- 100: intravascular blood pump
- 300: guidewire
- 302: guidewire body

## Claims

1. Sensor carrier (10, 310) configured to be attached to an elongated medical device (100, 300) comprising:
a sensor (12) having a sensor longitudinal axis (SLA),
a base member (14, 314) having a central longitudinal axis (CLA) and an attachment portion (16) for receiving the sensor (12), the sensor (12) being configured to be positioned in the attachment portion (16),
wherein the sensor longitudinal axis (SLA) is non-parallel with respect to the central longitudinal axis (CLA) of the base member (14).

2. Sensor carrier (10, 310) according to claim 1, wherein the sensor longitudinal axis (SLA) of the sensor (12) intersects with the central longitudinal axis (CLA) of the base member (14, 314).

3. Sensor carrier (10, 310) according to any one of the preceding claims, wherein the base member (14, 314) comprises a magnetically permeable material or is composed of a magnetically permeable material, in particular a plastic material, preferably PEEK (Polyether ether ketone).

4. Sensor carrier (10) according to any one of the preceding claims, wherein the base member (16) comprises an axial through hole (18) concentric with the central longitudinal axis (CLA), and wherein the axial through hole (18) comprises the attachment portion (16).

5. Sensor carrier (10) according to any one of the preceding claims, wherein the sensor carrier (10) is a teardrop (20) of a blood pump (100, 200), in particular of an intravascular blood pump.

6. Sensor carrier (10) according to claim 5, wherein the sensor carrier (10) further comprises an elastic extension (22) with a pigtail (24) having a tip (26), wherein the sensor longitudinal axis (SLA) points to the tip (26) of the pigtail (24).

7. Sensor carrier (10) according to claim 6, wherein the elastic extension (22) comprises an opening (28) for receiving a guidewire (30), the guidewire (30) being configured to elastically reel and unreel the pigtail (24).

8. Sensor carrier (10) according to claim 7, wherein the opening (28) is radially spaced from the central longitudinal axis (CLA) of the base member (14).

9. Sensor carrier (10) according to any one of the preceding claims, wherein the sensor carrier (10) further comprises a coupling element (32) attached to the base member (14), the coupling element (32) being configured to be attached to a suction head (34) disposed at a distal end of a cannula (36).

10. Sensor carrier (10) according to claim 9, wherein the coupling element (32) is made of metallic material, in particular of stainless steel, titanium or platinum.

11. Blood pump (100) comprising:
a sensor carrier (10) according to any one of the preceding claims 1 to 10,
a cannula (36), and
a suction head (34) fixed to the cannula (36), wherein
the sensor carrier (10) is coupled to the suction head (34).

12. Blood pump comprising:
a sensor carrier (210) including a sensor (12),
a cannula (36), and
a suction head (34) fixed to the cannula (36), wherein
the sensor carrier (210) is coupled to the suction head.

13. Blood pump (100) according to claim 11 or 12, wherein at least one cable (38) or at least one lead is connected to the sensor (12) and wherein the cable (38) is helically guided along and/or within the cannula (36).

14. Blood pump (100) according to claim 13, a coiled stabilizing structure (42) is provided along and/or within the cannula (36), wherein the at least one cable (38) is guided in-between the coiled stabilizing structure (42).

15. Guidewire (300) for introducing a blood pump into a patient's body comprising a sensor carrier (310) according to any one of claims 1 to 3.
